# EUROPEAN PATENT SPECIFICATION

(11) **EP 1 224 328 B1**
(45) Date of publication and mention of the grant of the patent: **26.06.2013**
(21) Application number: 00971618.4
(22) Date of filing: 27.10.2000
(51) Int. Cl.: C12Q 1/68

(54) **GENETIC BIOSENSORS USING CHEMILUMINESCENCE**
GENETISCHE BIOSENSOREN WELCHE CHEMILUMINESZENZ VERWENDEN
BIOCAPTEURS GENETIQUES A CHIMILUMINESCENCE

(30) Priority: 28.10.1999 GB 9925491
(43) Date of publication of application: 24.07.2002
(73) Proprietor: University College Cardiff Consultants Limited, Cardiff CF24 0DE (GB)
(72) Inventor: CRYER, Jennifer, Penarth South Glamorgan CF64 3HZ (GB); KILLE, Peter, Cardiff CF2 4LH (GB); MORBY, Andrew, Paul, Cardiff CF14 4BQ (GB); BROWN, Richard, Charles, Cardiff, CF1 9EP (GB)
(74) Representative: Fyles, Julie Marie
(86) International application number: PCT/GB2000/004168
(87) International publication number: WO 2001/031059

(56) References cited:
- WO-A-94/01584
- WO-A-94/17208
- WO-A-96/13612
- WO-A-97/27317
- WO-A-97/48823
- US-A- 5 283 174
- US-A- 5 399 491
- US-A- 5 643 730
- EMBER ISTVAN ET AL: "Early effects of cyclosporin A on in vivo oncogene expression." ANTICANCER RESEARCH, vol. 14, no. 3A, 1994, pages 1095-1096, XP001063995 ISSN: 0250-7005
- EMBER ISTVAN ET AL: "Effect of ABVD therapeutic protocol on oncogene and tumor suppressor gene expression in CBA/Ca mice." ANTICANCER RESEARCH, vol. 18, no. 2A, March 1998 (1998-03), pages 1149-1152, XP001063994 ISSN: 0250-7005
- MORGAN M B ET AL: "Assessing coral stress responses at the level of gene expression." AMERICAN ZOOLOGIST, vol. 39, no. 5, 1999, page 122A XP001064029 Annual Meeting of the Society for Integrative and Comparative Biology.;Atlanta, Georgia, USA; January 04-08, 2000 ISSN: 0003-1569
- RAJEEVAN MANGALATHU S ET AL: "Chemiluminescent analysis of gene expression on high-density filter arrays." JOURNAL OF HISTOCHEMISTRY AND CYTOCHEMISTRY, vol. 47, no. 3, March 1999 (1999-03), pages 337-342, XP001064022 ISSN: 0022-1554
- ROCKETT JOHN C ET AL: "Application of DNA arrays to toxicology: Workshop entitled "Application of Microarrays to Toxicology;Research Triangle Park, North Carolina, USA; January 7-8, 1996; US Environmental Protection Agency." ENVIRONMENTAL HEALTH PERSPECTIVES, vol. 107, no. 8, August 1999 (1999-08), pages 681-685, XP001064042 ISSN: 0091-6765
- NELSON N C ET AL: "DETECTION OF ACRIDINIUM ESTERS BY CHEMILUMINESCENCE" NONOSOTOPIC PROBING BLOTTING AND SEQUENCING, XX, XX, 1995, pages 391-428, XP000602666

## Description

This invention relates to methods and kits for determining the toxicological or pharmacological impact of chemicals, mixtures of chemicals or physical, chemical and/or biological conditions, hereinafter referred to as external factors, on living organisms or cells, and thus also for detecting or assaying specific chemical analytes or mixtures thereof. Such chemicals, chemical mixtures or external factors may exert a biological effect in terms of directly or indirectly modifying animal or plant cell function. The invention relies on the ability of living cells to produce a genetically mediated response following exposure to a chemical, mixture of chemicals or other external factor. Thus, within the environment of the organism or cell there may be present a component, plurality of components or alteration in the amounts of said components that results in a change in levels of specific messenger ribonucleic acid (mRNA) sequences in the cell relative to the unexposed or "normal" state. The change in mRNA production is detected by the binding of a complementary oligonucleotide probe labelled with a chemiluminescent molecule or a component of a chemiluminescent reaction. Similarly exposure of the organism or cell to other external factors such as, for example, heat, ultra-violet radiation, x-ray radiation may also result in changes in the levels of transcribed mRNA.

### Background of the Invention

The detection of alterations in the chemical components of the environment and their possible effects on the ecosystem may be undertaken using several standard bioassay procedures using living organisms [OECD (1984) *Guidelines for testing chemicals: Health Effects.* Organisation for Economic Cooperation and Development, Paris; US Environmental Protection Agency (1985) *Toxic Substances Control Act Test Guidelines: Final Rules. Fed Reg 40 CFR 798.2250, 2650, 2675, 3260, 3330, 4350, 4900;* European Community (1985) EEC Directive 79/831. Annex V., Part C: Commission of the European Communities, Doc EUR 9360 EN; Japanese Ministry of Agriculture, Forestry and Fisheries (1985) *Testing Guidelines for Toxicity Studies 59* Agricultural Chemical Laws and Regulations, Japan].

Living organisms and cells are sensitive to changes in their normal environment such that any change in the environment may lead to biological effects such as physiological changes in the organism or cell. Such changes have a biochemical basis and frequently result in increased or decreased rates of particular biochemical reactions, or even result in the genesis of reaction pathways not normally present (or active) in a given organism or cell type. The mechanisms of these physiological changes frequently contain a genetic component in that the environmental change causes part of the genome, or associated pathways, of the organism to be activated or deactivated. Ultimately, this leads to a physiological change involving the modulation of the biosynthesis of a protein such as, for example, an enzyme. This process involves signal transduction pathways that modulate the ability of the RNA polymerase complex to transcribe specific segments of the genome of the organism or cell as a consequence of changes in its environment. These signal transduction pathways work through direct or indirect interactions of the chemical, mixture of chemicals or other external factors with specific proteins which influence the activity of the transcription complex at defined nucleic acid sequences within the genome of the cell. The gene consists of a responsive element capable of sensing the environmental change and a reporter element which codes for the transcription of mRNA. Thus the presence and amount of an analyte in the environment of the organism or cell will influence activation of a gene such that, under the influence of RNA polymerase, part of its sequence is transcribed to yield a molecule of mRNA. Further the analyte may also affect the transcription process at parts of the biochemical pathway subsequent to initial gene activation. Moreover, the chemical or other external factor may cause down-regulation of mRNA transcription which may be present as part of the normal functioning of the biochemical pathway concerned. In the case of specific regulation subsequent to exposure, the amount of mRNA transcribed will generally depend on the amount or degree of exposure to the chemical, mixture of chemicals or other external factors. Normally, the mRNA is translated to yield a protein which may or may not have a physiological function in the organism or cell though pathways involving genetically engineered components may result in the formation of mRNA which does not result in the production of a translated protein.

Classically, the elucidation of the effects of exposure relied on monitoring the possible gross effects on the organism or cell from a holistic standpoint, for example death. More recently, the biochemical changes themselves have been monitored by measurement of translated proteins, enzymes or enzyme products. The detection or assay of environmental change by this means is thus crude and of limited utility.

### Description of the Prior Art

There exist in the literature numerous reports of the use of biochemical markers within cells or organisms to monitor the exposure of organisms to, for example, environmental contaminants such as heavy metals, pesticide residues, plasticisers or detergent residues [P Kille et al. (1992) Aquat. Toxicol. 22, 279-286; W Baturo et al. (1996) Environmental Toxicology and Chemistry 15, 771-781; T Stahlschmid et al. (1997) Environmental Science and Pollution Research 4, 155-162.]. An example of such an approach is that which is used to establish the presence of water-borne environmental contaminants that have estrogenic activity. The protein vitellogenin is not normally present in male fish. However, it is known that this protein is produced by male fish which have been chronically exposed to estrogenic substances or to environmental contaminants that mimic estrogenic activity such as certain detergent residues [S Jobling et al. (1993) Aquat. Toxicol. 27, 361-372; CE Purdom (1994) Chemistry and Ecology 8, 275-285; CB Lazier et al. (1993) In: Biochemistry and Molecular Biology of Fishes. Ch. 19, Vol. 2. Hochanchka and Mommsen (eds), Elsevier Science Publishers.]. Thus it is possible to introduce a normal male fish to the suspected contaminated water and subsequently subject a sample of tissue or body fluid from the fish to an assay for vitellogenin. Such a test would normally be performed using SDS protein gel electrophoresis [UK Laemmli (1970) Nature (London) 227, 680-685] of blood plasma or using an immunoassay [S Jobling et al *(loc cit);* CE Purdom et al (*loc cit*); CB Lazier et al (*loc cit*)]. In this way, the presence of vitellogenin would be indicative of the presence of a contaminant having estrogenic activity.

Many methods have involved the assay of a gene product, i.e. a protein. However, such methods are time-consuming and of limited utility. In attempts to overcome such disadvantages, there has been reported means by which a range of different genetic techniques have been used to identify changes in the pattern of gene transcription caused by environmental or disease changes [CG Sagerstrom et al (1997) Ann. Rev. Biochem. 66, 751-783]. Many genetic elements responsive to chemical exposure ("stress genes") of cells and organisms are already known. This has, for example, been demonstrated using earthworms exposed to heavy metal contaminated soil [SR Sturzenbaumn (1998) Applied Soil Ecology 9, 495-500; SR Sturzenbaumn et al (1998) In: Advances in Earthworm Ecotoxicology, Sheppard et al (eds), SETAC Press, pp 215-224]. Though this technique does not rely on the determination of translated gene products, it suffers from the constraints of existing techniques used routinely for analysing mRNA levels such as Northern blotting, RNase protection assays and RT-PCR [J Sambrook et al (1989) Molecular Cloning: A Laboratory Manual, 2nd Ed., Cold Spring Harbor Laboratory, Cold Spring Harbor, New York; CT Wittwer et al (1997) Gene Quantification*].* US 5,643,730 discloses a process for indirectly detecting the presence and measuring the quantity of specific mRNA sequences in cells, comprising the time-consuming steps of producing one or more cDNA sequences from mRNA by removing culture medium from cultured cells, lysing the cells, allowing the lysate to cool, producing cDNA, amplifying the cDNA and only then proceeding with the process of detecting the presence of cDNA. Similarly, Jessen-Eller et al describes an indirect method for determining mRNA expression which involves RT-PCR, incorporating *in vitro* transcription, followed by the use of a number of pre-defined primer sequences labelled with ruthenium II chelate in order to amplify target nucleic acid for analysis [Jessen-Eller et al (1994) Biotechniques. 17(5), 962-973]. Alternatively, WO/9748823 discloses an indirect assay system for screening nutraceticals capable of modulating expression of genes associated with undesirable conditions, measured by level of mRNA of a reporter gene as assayed using conventional nucleic acid dot-blot techniques. This involves the laborious and indirect techniques of running RNA samples on a gel in order to separate fragments of different sizes and identifying the fragments by using a hybridisation probe.

The utility of these procedures can be assessed using a number of criteria including: the degree of technical knowledge needed to execute the procedure; inclusion of hazardous reagents i.e. radiolabelled nucleotides; time taken to perform the assay; cost; sensitivity; specificity and the accuracy of measurement i.e. whether the result is qualitative or quantitative. The most widely used technique is Northern blotting which 1) requires a highly trained operator, 2) utilises radioisotopes, 3) requires 2-3 days, 4) is expensive, 5) requires 10-20 µg of total RNA. Moreover, cross reactivity can occur between isoforms and the procedure provides only qualitative results.

RNase protection assays exhibit greater specificity and require ~10 fold less starting material but are more time consuming, require the preparation of a radioactive probe and involve increased cost. Although, radioactive detection can be substituted with chemiluminescence detection, these procedures utilise indirect ligand binding of chemicals such as biotin which have been covalently linked to the DNA probe. Due to the indirect nature of this detection these procedures require extra steps and reagents and are prone to background interference due to the signal amplification required to perform the detection. The limitations on utility for all the present techniques are the high skill levels required of the operator, the time taken to perform the procedures, the cost and the poor level of reproducibility of the results.

The use of direct chemiluminescent-labelled oligonuceotide probes has been described for the quantification of ribosomal RNA, using a homogenous assay format utilising oligonucleotide probes labelled with acridinium esters which are explicitly said to be unstable and so limited in their application [US 5 283 174; US 5 399 491; Nelson et al IN: Nonisotopic probing, blotting and sequencing (1995), pages 391-428]. However, such methods have only been demonstrated as a means of detecting bacteria responsible for causing infectious diseases. Here, reliance is placed on the ability of such methods to detect the large quantities of ribosomal RNA (rRNA) present in the bacterium.

We have developed novel methods by which changes in the level transcription of mRNA of organisms or cells or other transcription products exposed to chemicals, mixtures of chemicals or other external factors are monitored by the use of direct chemiluminescent-labelled oligonucleotide probes. The teachings embodied herein permit for the first time the development of reagent kits and methods for the near real-time assessment of the possible toxicological or pharmacological activity of chemicals, mixtures of chemicals or other external factors on living organisms in a rapid, sensitive and reproducible manner.

### Summary of Invention

According to one aspect of this invention, there is provided a method for the detection and quantification of chemicals, mixture of chemicals or other external factors which comprises:
a. exposing to said chemical, mixture of chemicals or other external factor a non-human organism, cell or genetic assembly comprising a gene responsive to said chemical, mixture of chemicals or other external factor such that a change occurs in the level of transcribed mRNA as a result of activation or deactivation of said gene;
b. directly binding to said transcribed mRNA an oligonucleotide probe which is complementary to at least a part of said mRNA produced and which is labelled with a chemiluminescent acridinium ester; and
c. quantifying said mRNA using a chemiluminescent signal produced by said probe..

Whilst mRNA has been particularly identified as a useful target transcription product in the present teachings, it is taught herein that other transcription products can be detected and/or quantified in the same way.

It is taught herein that the chemiluminescent molecule is a chemiluminescent acridinium salt. It is also taught that the chemiluminescent molecule may be a chemiluminescent salt selected from the group pyridinium, phenanthridinium, benzacridinium or quinolinium. Alternatively it is disclosed that said chemiluminescent label may be a chemiluminescent acridan.

Still further, it is disclosed that the chemiluminescent label may be a chemiluminescent acridine.

Furthermore it is taught the oligonucleotide may be labelled with a substance capable of taking part in a chemiluminescent reaction.

Preferably, the chemical reactivity of the molecule comprising the oligonucleotide label will be different when the said oligonucleotide is bound to a complementary target sequence when compared to the chemical reactivity when not so bound.

A plurality of different labelled oligonucleotide probes may be used to detect the presence or absence of complementary target mRNA sequences in the same analytical sample.

The gene may be a recombinant molecule in a prokaryotic cell comprising at least one each of a response element functionally linked to a reporter element.
2. In another aspect, this invention provides a kit for the direct quantification of a chemical, mixture of chemicals or other external factors. The said kit is used together with non-human organisms, cells or genetic assemblies which have previously been exposed to a chemical, mixture of chemicals or other external factor which comprises:
   a. at least one oligonucleotide probe complementary to at least a part of the mRNA produced by the said non-human organisms cells or genetic assemblies in response to said chemical, mixture of chemicals or other external factors, and labelled with a chemiluminescent acridinium ester; and
   b. non-human organisms, cells or genetic assemblies comprising either:
      i. a gene responsive to said chemical, mixture of chemicals or other external factor to produce a change in the level of transcribed mRNA as a result of activation or deactivation of said gene; or
      ii. a transcription pathway responsive to said chemical, mixture of chemicals or other external factor to produce a change in the level of transcribed mRNA as a result of exposure of said non-human organisms, cells or genetic assemblies to said chemical, mixture of chemicals or other external factors.

Accordingly, the present invention relates to the determination of the toxicological or pharmacological impact of chemicals, mixtures of chemicals or other external factors on organisms or cells and thus also to the detection of and quantification of such chemical analytes or conditions by the use of the genetically-mediated response of organisms or cells exposed to such chemicals or changes in the concentrations thereof or changes in the conditions of other external factors. This response is monitored by the use of oligonucleotide probes that are complementary to defined sequences of mRNA or other transcription products transcribed from genes. The activity of the genes or transcription pathway may be altered in the presence or absence of the said chemicals, mixtures of chemicals or other external factors. The binding of these oligonucleotide probes is in turn monitored by previously labelling them with a chemiluminescent ester in such a way that the light emission from the labelled probe is a measure of the extent of hybridisation of the probe with its complementary target sequence. In this way, the intensity of light emission is proportional to the amount of transcribed sequence, which in turn is a measure of the activity of a particular gene or transcription pathway responsive to the exposure of the organism, cell or genetic assembly to the chemical, mixture of chemicals or other external factors. This invention has utility in several fields of application such as, but not restricted to, the following:
1) determination of the toxicity of chemicals which may be released into the biosphere,
2) detection and/or quantitation of chemical contaminants in the environment,
3) determination of the toxicity and/or therapeutic activity of pharmacological preparations
4) assessment of possible adverse effects of personal care products.

Though the teachings embodied herein are particularly directed to the assessment of possible deleterious consequences of the exposure of the organism or cell to a given chemical species or conditions, it can be appreciated that the same approaches can be used in situations where beneficial consequences have a genetically mediated component. Moreover, the gene assemblies involved in these mechanisms can be isolated and/or engineered such that the transcribed mRNA or other transcription product can be produced completely *in vitro* without any requirement for the use of intact organisms or cells. The use of these methods offers advantages in terms of simplicity and speed over other methods thus for the first time allowing real-time determinations to be performed. The use of such chemiluminescent labelled oligonucleotides to monitor gene activation in this way is both novel and surprising since there is no teaching that suggests the utility of this type of end-point for anything other than the detection of infectious organisms in biological specimens.

### Description of Preferred Embodiments

In a preferred aspect of the invention, an organism or cell is identified which possesses a gene which is responsive to a specific environmental parameter such as a chemical or group of chemicals which needs to be detected or quantified. One skilled in the art readily appreciates the means of identification of such genes. An example of this kind of approach is differential display.

The chemicals of interest are exemplified by, but not limited to, environmental pollutants such as heavy metals (mercury, cadmium etc.), pesticide residues (organophosphorus compounds etc.), herbicide residues (triazines, glyphosate etc.) polyaromatic hydrocarbons, polychloronated biphenyls and other synthetic or naturally occurring compounds. Firstly, the gene responsive to the analyte or analytes of interest is identified and characterised using methods already established and known to those skilled in the art. Secondly, an oligonucleotide probe is synthesised by established methods such that its sequence is complementary to a region of a specific mRNA molecule that is transcribed from a gene responsive to exposure of the non-human organism or cell system to the analyte of interest. Thirdly, the oligonucleotide probe is labelled with a chemiluminescent este. The light emission from the chemiluminescent ester is indicative of the hybridisation of the probe with the specific mRNA produced in response to the gene activation. Alternatively, the chemiluminescent labelled oligonucleotide probe can be used to detect changes in mRNA levels due to exposure to chemicals, mixtures of chemicals or other external factors which do not *per se* affect gene regulation but affect other elements in the transcription mechanism such as RNA polymerase activity. In a particularly preferred aspect, use is made of the property of hybridisation protection of chemiluminescent acridinium salts by double stranded nucleic acids as described in U.S. Patent 5 283 174. Here, a labelled oligonucleotide probe is allowed to hybridise to its exact complementary nucleic acid target and the reaction conditions are then changed (for example by elevation of temperature and/or pH) such that the chemiluminescent properties of the unhybridised or nonspecific hybridised labelled oligonucleotide are destroyed by hydrolysis of the chemiluminescent molecule. Since this hydrolysis does not occur when chemiluminescent oligonucleotide probe is hybridised to its exact complementary target sequence, then subsequent estimation of chemiluminescent activity is proportional to the amount of target sequence present. Based on the use of such chemiluminescent end-points disclosed in U.S. Patent 5 283 174 one skilled in the art would readily appreciate generally how to design, synthesise and use chemiluminescent oligonucleotide probes. There also exist in the literature descriptions of other chemiluminescent systems including, for example, pyridinium, phenanthridinium, benzacridinium and quinolinium salts; acridans and acridines. Ultimately, any chemiluminescent compound or component of a chemiluminescent reaction which has been proposed to have general utility as a label for an oligonucleotide probe may be used in the present context.

The present teachings demonstrate the surprising finding that the techniques of gene identification, mRNA targetting and chemiluminescent labelled oligonucleotide probe detection can be effectively combined in such a way that a method is produced which permits the rapid, cost effective, sensitive, detection and/or quantitation of a wide range of analytes by a method that can be easily performed. Moreover, the method is particularly desirable for the assessment of bioavailable contaminants which may have an adverse ecological effect. Such a method has not previously been described nor is it apparent that such a system as described could be developed.

It can be appreciated that the same technical principles taught herein can be applied to any situation in which an organism or cell elicits a genetic response as a result of exposure to a chemical or physical stimulus, said response resulting in the formation of nucleic acid to mRNA.

It is taught herein that changes in nucleic acid concentrations as a result of the influence of chemical or physical changes on other elements of the transcription pathway can be used. By way of example, it is known that the synthesis of transcribed sequences relies on the activity of particular enzymes. Monitoring the changes in the concentration of transcript therefore also allows a measure of the activity of these enzymes to be obtained. This method is particularly advantageous for assessing the activity of putative pharmacological compounds suspected of affecting the activity of such enzymes. Examples of such enzymes are polymerase and reverse transcriptase. In particular such methods are useful for the screening of compounds having possible utility as antibacterial or antiviral agents as a consequence of their effect on enzymes involved with replication.

The identification of chemicals having pharmacological activity is key to drug discovery programs. In particular, the advent of combinatorial synthesis requires the screening of large numbers of small quantities of chemical agents for pharmacological activity and for toxicity. Current methods are poorly adapted for such purposes and are complex, time-consuming and expensive. By contrast, the teachings disclosed herein permit cellular mRNA responses to be monitored in near real-time and allow simultaneous study of the activities of large numbers of putative therapeutic agents.

In a similar aspect, there is much concern as to the possible collateral toxic effects of newly-developed chemical species such as herbicides and pesticides on non-targetted organisms in the environment. Since toxic stress invariably results in changes in regulation of mRNA production in an exposed organism, it is possible to determine the toxicity of newly-developed chemical species using the teachings described herein.

In practical terms, established techniques can be used for the husbandry of organisms, maintenance of cells and isolation and/or engineering of *in vitro* genetic assemblies from which samples of mRNA or other transcription product are derived.

For reference purposes, the mRNA derived from a suitable "housekeeping" gene such as that corresponding to the protein actin is subjected to the same procedure as the stress gene mRNA. Such housekeeping genes, which do not respond to the same stimuli as the responsive genomic elements being targetted, are chosen as controls for the method. Thus, in a particular example, extracts of organism target tissue or cell-lines can be prepared from non-exposed and putatively exposed organisms or cells and the mRNA contained therein analysed using the chemiluminescent labelled oligonucleotide probes of the desired sequences.

Extraction of nucleic acid from tissue homogenates or cell lysates can be performed according to published methods. A sample of the extracts is mixed in an appropriate buffer solution with a known quantity of chemiluminescent labelled oligonucleotide probe under appropriate conditions. Presence of target having a sequence complementary to the probe sequence results in hybridisation. According to the invention, the oligonucleotide is labelled with a chemiluminescent acridinium ester. Here, following the hybridisation procedure, the reaction mixture is exposed to alkaline conditions at a temperature of preferably in the range 20 to 80 degrees C, more preferably 55 to 65 degrees C. Under such conditions, unhybridised probe is selectively hydrolysed such that its chemiluminescent activity is lost and therefore the presence of chemiluminescence emission as measured in a luminometer is indicative of the presence of target. Appropriate procedures for performing hybridisation reactions and for the measurement of chemiluminescence are well-known to those skilled in the art. These procedures may be performed in separate parallel experiments using, respectively, oligonucleotide probes in one instance to the target of choice and in the other instance to actin mRNA as control. The finding in the sample from the exposed organism or cell-line of increased target relative to that expected for an organism or cell-line not exposed to the putative toxic chemical is indicative of the presence of a toxic stress. As an alternative to this approach, there exists the possibility of performing the sample and control experiments contemporaneously in the same reaction environment. Here use is made of the ability to label different oligonucleotides respectively with chemiluminescent labels having distinct chemical or optical properties. Such procedures which, for example, involve the discrimination of chemiluminescent compounds on the basis of their reaction kinetics are already established [US Patent 5 756 709].

The use of prokaryotic cells is particularly advantageous in respect of the teachings described herein since it is possible to use current molecular biology techniques to create artificial "gene" sequences or constructs containing both the desired response element (promoter sequence) and a "reporter" sequence from which mRNA or other target nucleic acid is transcribed in response to the binding of the analyte of interest to the promoter. The creation of such assemblies is well-established and it is possible to link promoters to any nucleotide sequence capable of being transcribed regardless of whether or not the target nucleic acid so produced exists in nature and regardless of whether it is capable of yielding a translated protein.

In this context it is also possible to make use of biochemical assemblies without the need for intact prokaryotic cells. The use of *in vitro* transcription for example is well-established and it is therefore possible to establish the effects of changes in the chemical/physical environment of the biochemical system corresponding to a chosen transcription pathway. The biochemical system can be used to monitor effects at the level of gene regulation itself or at other levels in the transcription pathway such as the enzymes involved in transcription.

Moreover it is possible to engineer constructs with a plurality of response and reporter elements.

In order that the invention may be more fully understood, the following non-limiting examples are given for illustrative purposes.

### List of Figures

Figure 1 is a chart showing the results of an assay with mouse liver total RNA using acridinium ester actin probe with a negative control (marked "probe") and mouse actin mRNA in varying amounts;
Figure 2 is a chart showing an assay of mouse liver total RNA using mouse specific acridinium ester labelled probe in the presence of salmon actin mRNA. (Probe = negative control);

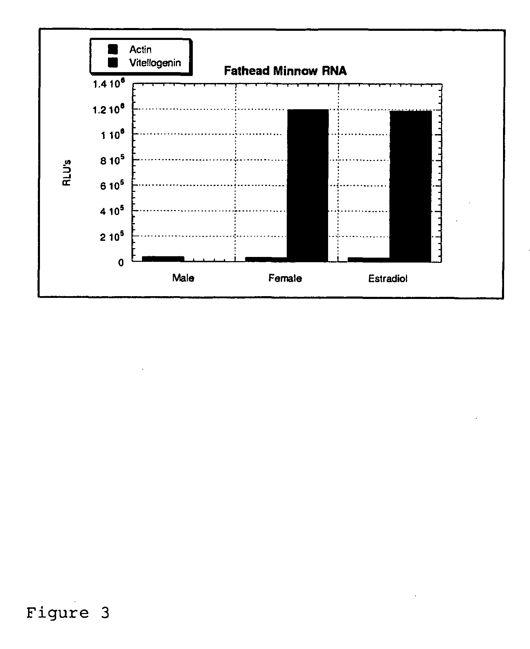

Figure 3 is a histogram showing Relative Light units (RLU's) measured with Fathead Minnow Actin mRNA and Vitellogenin mRNA acridinium-labelled probes in Fathead Minnow male, female and estradiol induced juvenile total RNA preparations;
Figure 4 is a chart showing induction of vitellogenin in fathead minnows after expose to 17β-estradiol. The bars represent mean values ± S.E.M. (n = 5). The data are expressed as fmol/µl mRNA equivalents (derived from a linear regression of a calibration curve using synthetic target oligonucleotide).
Figure 5 is a chart showing vitellogenin induction in fathead minnows following environmental exposure to 17β-estradiol. The bars represent mean values ± S.E.M. (n = 5). The data are expressed as fmol/µl mRNA equivalents (derived from linear regression of a calibration curve using synthetic target oligonucleotide).
Figure 6 is a schematic diagram of the pRZ-ES1 construct; and
Figure 7 is a histogram showing the detection of mercury response in genetically-modified E. coli using chemiluminescent acridinium-labelled oligonucleotide probe sequence complementary to lacZ mRNA.

### Examples

### EXAMPLE 1

### Preparation of acridinium labelled oligonucleotide probe to mouse actin RNA

The following oligonucleotide probe sequence to mouse actin RNA was synthesised using established methods.

### 5'-GGA GGA GCA AT*G ATC TTG ATC TTC-3' ( * indicates position of linker)

A non-nucleotide linker terminating in an amino group was introduced into the sequence during synthesis. The probe was labelled with 4-(2-succinimidyloxycarbonylethyl)phenyl-10-methylacridinium-9-carboxylate trifluoromethanesulphonate and purified using high performance liquid chromatography. The synthesis, labelling and purification of the probe was carried out as described in the literature ("Detection of acridinium esters by chemiluminescence", NC Nelson and MA Reynolds, in "Nonisotopic Probing, Blotting and Sequencing", (1995) Academic Press Inc., pp. 391-427; LJ Arnold and NC Nelson, US Patent 5 185 439; LJ Arnold et al. WO88/03173.

### EXAMPLE 2.

### Quantitation of mouse actin mRNA

Total RNA was prepared from mouse liver tissue using a commercially available method (Qiagen Rneasy Midi Kit) and eluted into sterile, pure water. The concentration of total RNA recovered was routinely 400 - 800 ug/ml as determined by optical absorbance measurement.

The assay was performed using triplicate determinations. Hybridisation reactions were carried out in 12 x 75 mm polystyrene tubes (Sarstedt, Cat. No. 55.476) and comprised 50ul double strength hybridisation buffer (0.1 mol/l lithium succinate, pH 5.2; 3 mmol/l EDTA; 3 mmol/l EGTA; 17% (w/v) lithium lauryl sulphate), 20fmol acridinium ester labelled oligonucleotide probe prepared according to Example 1 (complementary to mouse β-actin gene [Genebank accession no: X03672]) and a defined quantity of total RNA. The reaction volumes were made up to 100ul. Controls consisted of the following: blank samples (reagents only), negative control (all components except target nucleic acid), positive control (all components but with the total RNA replaced by a known amount of standard oligonucleotide complementary to the probe sequence). The reaction tubes were stoppered and incubated for 30 minutes at 60°C.

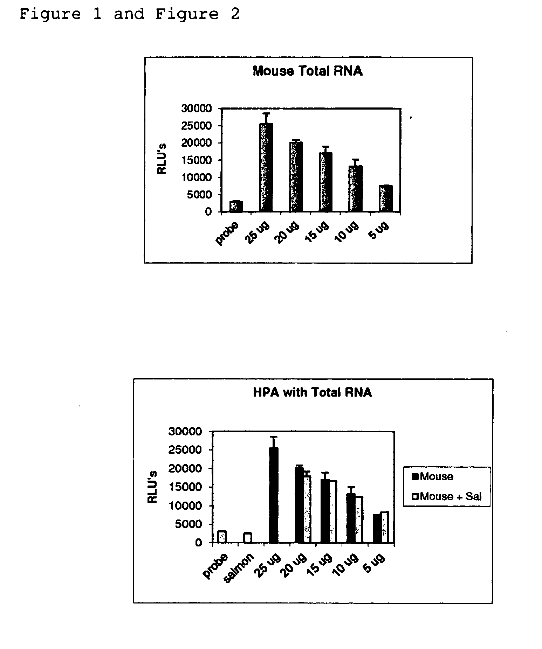

300ul hydrolysis buffer (190mmol/l sodium borate, pH 7.6; 5% v/v Triton X-100) were added to each tube and the reactions incubated at 60°C for a further 10 minutes. Immediately following incubation, the tubes were cooled on ice for 1 minute and then left at room temperature for a further 1 - 2 minutes. Chemiluminescence was measured as relative light units (RLU) in a luminometer (Stratec Lumino) for 5 seconds following sequential injection of 200ul each of Pace 2 Detection Reagents I and II (Gen Probe). The results are set out in Table 1 below and represented graphically in Figure 1.

Table 1: Chemiluminescence response for mouse actin mRNA. RLU values represent the mean of triplicates with subtraction of blank.

| RNA (ug) | RLU | Std. Dev. |
|---|---|---|
| 25 | 25489 | 3065 |
| 20 | 20104 | 743 |
| 15 | 16991 | 1961 |
| 10 | 13258 | 1932 |
| 5 | 7618 | 120 |

### EXAMPLE 3.

### Specificity of assay for mouse actin mRNA

The mouse actin acridinium ester labelled oligonucleotide probe described above possessed a single base mismatch when compared to the analogous salmon actin sequence (Genebank accession no: AF012125). This mismatch affects the hybridisation kinetics such that the system is specific for total complementarity. This specificity is demonstrated by adding an amount of salmon actin sequence (prepared from salmon liver tissue in a manner described for the preparation of mouse actin RNA described in Example 2) equivalent to the amount of mouse actin sequence present. The assay was performed in the same way as for mouse actin RNA, and the results are presented graphically in Figure 2. It can be seen that the presence of salmon actin sequence caused no interference in the detection of the mouse actin mRNA in those reactions where it was added.

| | |
|---|---|
| | AE |
| | ↓ |
| Probe | 5'-GGA GGA GCA ATG ATC TTG ATC TTC- 3' |
| RC-Actin mouse | 5'-GGA GGA GCA ATG ATC TTG ATC TTC- 3' |
| RC-Actin salmon | 5'-GGA GGG GCG ATG ATC TTG ATC TTC- 3' |

| | |
|---|---|
| * Note: gene sequences are reverse complement. i.e. antisense strand. AE = acridinium ester. | |

### Example 4

### Earthworm actin gene

Total RNA was isolated from whole earthworm (Lumbricus rubellus) tissue. The method differed slightly from that for the isolation of total RNA from liver tissue as the former is highly collagenous and requires a more stringent method. Here total RNA was isolated using TRI reagent (Sigma Chemical Co.) following established methods. The RNA pellet obtained from this process was dissolved in sterile, pure water and washed through a Qiagen RNeasy Maxi column according to manufacturer's instructions. The assay for earthworm actin RNA was carried out in the same way as described in Example 2 except that an acridinium labelled probe was used which was complementary to the earthworm actin gene (Genebank accession no: Y09623). The results are given in Table 2 below.

Table 2: Chemiluminescence response for earthworm actin RNA. RLU values represent the mean of triplicate determinations.

| RNA (ug) | RLU | Std. Dev. |
|---|---|---|
| 1.5 | 4345 | 131 |
| 3.1 | 5125 | 259 |
| 6.1 | 7910 | 1221 |
| 12.3 | 13836 | 1184 |
| 18.4 | 19133 | 1474 |
| 24.6 | 24745 | 65 |

### Example 5

### Measurement of Vitellogenin mRNA in Fathead Minnow (Pimephales promelas)

The production of vitellogenin, an estrogen-dependent yolk protein precursor, can serve as a valuable biomarker for exposure to estrogen in oviparous vertebrates. This biomarker can be used *in vitro* (hepatocyte cultures) and in *in vivo* studies. Although vitellogenin is normally present only in the plasma of female fish, males do have the vitellogenin gene and exposure of male fish to environmental estrogens can trigger expression of the gene. The fathead minnow, *Pimephales promelas,* is one of the most widely used fish species in ecotoxicology.

Acridinium labelled oligonucleotide probes (5' CGG GCA AT*G ACA GCA AAA ACA GG-3'; * = acridium ester) designed to hybridise with fathead minnow vitellogenin mRNA were used to measure the expression of the gene in male, female and estradiol-induced juvenile fathead minnows. Actin expression was also measured for each sample, as actin is widely accepted as being a ubiquitously expressed gene, and can thus be used as a control.

The following experimental procedure was adopted:
1. Total RNA was extracted using established procedures with TRI Reagent (Sigma Chemical Co.) from the following:
   Adult Male Fathead Minnow
   Adult Female Fathead Minnow
   Juvenile Estradiol-Induced Fathead Minnow
2. HPA Format:
   The following reaction mixture was prepared in 12mm x 75mm Sarstedt tubes for each sample to be measured.
   Duplicate samples were prepared for each measurement.
      50 µl Double strength hybridisation buffer
      10 µl acridinium labelled probe (- 0.1 pmol)
      X µl Total RNA (~ 6 µg)
      Y µl Sterile water

Where X and Y are selected to give a total reaction volume of 100 µl.

### Hybridisation:

All tubes were incubated in a water bath at 60°C for 30 minutes.

### Hydrolysis:

300 µl of hydrolysis buffer (190mM sodium borate, pH 7.6; 5% v/v Triton X-100) were added to each tube.

All tubes were incubated for a further 10 minutes in a circulating water bath at 60°C.

All tubes were then cooled in an ice bath for 2 minutes, then at room temperature for 2 minutes, before being measured.

### Detection:

Chemiluminescence of each replicate was measured in a luminometer (Stratec Biomedical Systems) using a 200ul injection of Detection Reagents I and II (Gen Probe Inc., San Diego, U.S.A) and a 5 second measurement time.

### Example 6

### Measurement of Vitellogenin mRNA expression in fathead minnows following intra-peritoneal (IP) exposure to 17β-estradiol

IP exposure:17β-Estradiol (5mg/kg) was administered by intraperitoneal injection on day zero. Five fish were sampled on days 1, 3, 5 and 7. Control fish (no estradiol) were also sampled.

Assay: Vtg mRNA was extracted and measured as outlined in Example 5; approximately 15 µg of total RNA was used per measurement.

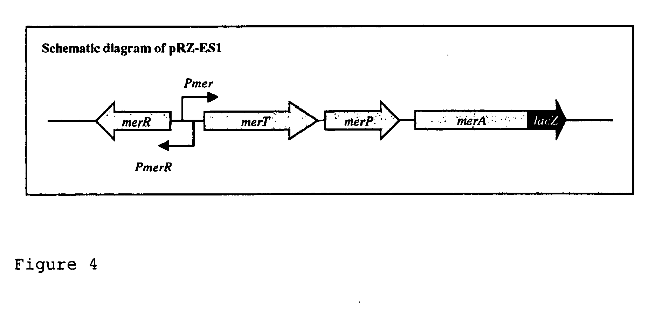

Samples were measured relative to a set of target calibration standards complementary to the labelled probe sequence (target concentrations = 0.1, 0.5, 1.0, 5.0, 10 & 50 fmol/ 10µl). The results are shown in Figure 4.

### EXAMPLE 7

### Vitellogenin mRNA induction following environmental 17β-estradiol exposure

Environmental exposure: Juvenile fathead minnows (weight range = 0.30 - 1.0 g) in 40 litre aquaria were exposed to 100ng/L of 17β-estradiol via a flow-through dosing system. Five fish were sampled on days 0, 7, 14 & 21.

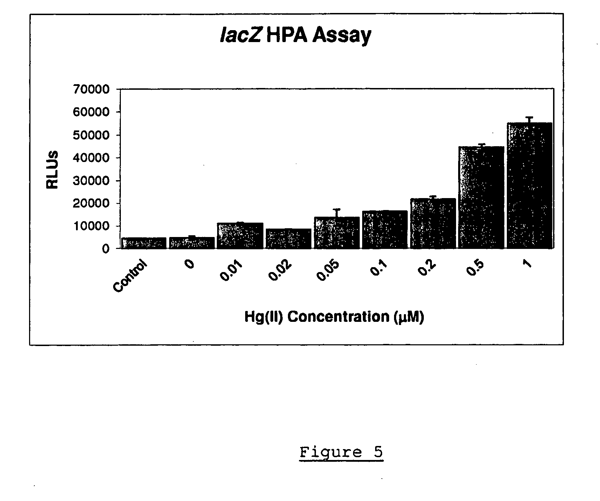

Assay: Vtg mRNA was extracted and measured as outlined in Example 6; approximately 15 µg of total RNA was used per measurement. The results are shown in Figure 5.

### EXAMPLE 8.

### Detection of mercury using genetically modified E coli and chemiluminescent hybridisation assay.

Plasmid pRZ-ES1 contains genes that encode a Hg(II) transport system, a Hg(II)-responsive transcriptional regulator, and its cognate promoter which directs lacZ transcription. A schematic diagram of the genes and promoters present in pRZ-ES1 (as described in Peter A. Lund et al (1987) Gene 52, pp207-214) is shown in Figure 6. The following steps were carried out:

### Preparation of competent E. coli

An aliquot (5 ml) of *E*. *coli* TG2 was used to inoculate a solution of (10g/l bacto-tryptone, 5g bacto-yeast extract, 10g/l sodium chloride, pH 7.0 ("LB" solution, 100 ml) and incubated with shaking (37°C, 225 rpm) until the OD₆₀₀ reached 0·45-0·60 (approximately 3 hrs). The culture was transferred to two 50 ml conical tubes and centrifuged (3,000 rpm, 5 mins). The resulting pellet was resuspended in cold CaCl₂ (50 mM, 25 ml) and chilled on ice (20 mins), followed by centrifugation at 4°C (3,000 rpm, 5 mins). The pellet was resuspended in cold CaCl₂ (50 mM, 10 ml) and chilled on ice (1-6 hrs). The cells were then ready to use or alternatively were stored as a glycerol stock by adding sterile glycerol (40%, 10 ml) and storing in aliquots (200 µl) at -70°C. When the cells were required they were thawed slowly on ice (15-30 mins).

### Transformation of competent E. coli

Competent TG2 bacteria (200 µl) were transferred into a chilled 15 ml conical tube. pRZ-ES1 DNA (approximately 10-50 ng) was added to the cells and swirled gently. The tube was chilled on ice (30 mins) followed by heat shock (42°C, 3 mins). The tube was immediately cooled on ice (2 mins) prior to adding a solution of 16g/l bacto-tryptone, 10g/l bacto-yeast extract, 5g/l sodium chloride ("2TY", 800 µl, preheated to 42°C). The bacteria were incubated with shaking (37°C, 225 rpm, 1 hr). An aliquot (50-100 µl) of the transformation mixture was plated onto "LB"-Agar ("LB" solution plus 15 g/l bacto-agar) plates containing kanamycin antibiotic and incubated (37°C, 18 hrs). If necessary the cells were concentrated by centrifugation (1,000 rpm, 10 mins) and re-suspended in "2TY" (50 µl) prior to plating.

### Induction of bacteria with Hg(II)

A single colony containing the pRZ-ES1 plasmid was picked and used to inoculate "2TY" media (5 ml). The culture was incubated (37°C, 18 hrs). A ten-fold dilution of the overnight culture in "2TY" was incubated for 2 hours at 37°C. A further ten-fold dilution in "2TY" was inoculated with various HgCl₂ concentrations, while a second dilution with no HgCl₂, was used as a control. After inducing for an appropriate timescale total RNA was prepared using a Qiagen RNeasy kit.

### Isolation of bacterial total RNA

RNA isolation was performed using RNeasy kit (Qiagen) according to the manufacturer's instructions.

### Hybridisation Protection Assay (HPA)

LacZ-4 was used since the corresponding probe to its transcribed mRNA had previously been determined to perform better in HPA assays than LacZ-2 since greater sensitivities and shorter hydrolysis times could be achieved. The lacZ HPA Assay conditions used were as follows.

The following reaction mixture was prepared in a microcentrifuge tube and incubated (60°C, 30 min).
15 µl Double Strength Hybridisation Buffer 1 µl Acridinium labelled-probe (0.05 - 0.1 pmol; 4 - 5 x 10⁶ relative light units) 10 µl Target from step 4 (0.5 - 1.0 pmol) 4 µl H₂O

After incubation Single Strength Hybridisation Buffer (270 µl) was added and an aliquot of the diluted mixture (10 µl) was transferred into a 12 x 75 mm Sarstedt tube and placed in a water bath rack. For time zero measurements, 200 µl Detection Reagent I (Gen Probe) was added, followed by the addition of 100 µl of Hydrolysis Buffer. Chemiluminescence was measured in the luminometer as described below. To the replicates in the rack Hydrolysis Buffer (100 µl) was added and mixed thoroughly. The tubes were placed immediately in a water bath at 60°C for the required hydrolysis time (10 mins). At the desired time points one set of replicates was removed from the water bath and Reagent I (200 µl) was added to each. Tubes were shaken to ensure thorough mixing. Tubes were then placed on ice until ready to measure the chemiluminescence. The tubes were placed at room temperature for approximately 1 min prior to measurement in a luminometer using a single injection of Reagent II (Gen Probe) (200 µl and a 5 second measurement time. The results are shown in Figure 7.

## Claims

1. A method for the quantification of a chemical, mixture of chemicals or other external factors, which comprises:
a. exposing to said chemical, mixture of chemicals or other external factor a non-human organism, cell or genetic assembly comprising a gene responsive to said chemical, mixture of chemicals or other external factor such that a change occurs in the level of transcribed mRNA as a result of activation or deactivation of said gene;
b. directly binding to said transcribed mRNA an oligonucleotide probe which is complementary to at least a part of said mRNA produced and which is labelled with a chemiluminescent acridinium ester; and
c. quantifying said mRNA using a chemiluminescent signal produced by said probe.

2. A method for the quantification of a chemical, mixture of chemicals or other external factors, which comprises:
a. exposing to said chemical, mixture of chemicals or other external factor a non-human organism, cell or genetic assembly comprising a transcription pathway responsive to said chemical, mixture of chemicals or other external factor such that a change occurs in the level of transcribed mRNA as a result of said exposure;
b. directly binding to said transcribed mRNA an oligonucleotide probe which is complementary to at least a part of said mRNA produced and which is labelled with a chemiluminescent acridinium ester; and
c. quantifying said mRNA using a chemiluminescent signal produced by said probe.

3. A method according to any of the preceding Claims in which the chemical reactivity of the molecule comprising the oligonucleotide probe label is different when the oligonucleotide probe is bound to the target mRNA sequence as compared with that of the said oligonucleotide probe label when the said oligonucleotide probe is not so bound.

4. A method according to any of the preceding Claims in which a plurality of different labelled oligonucleotide probes are used to detect the presence or absence of complementary target mRNA sequences in the same sample.

5. A method according to any of the preceding Claims, wherein the gene is a recombinant molecule in a prokaryotic cell comprising at least one each of a responsive element functionally linked to a reporter element.

6. A kit suitable for the method of any of claims 1-5 for the direct quantification of a chemical, mixture of chemicals or other external factors for use with non-human organisms, cells or genetic assemblies previously exposed to said chemical, mixture of chemicals or other external factors, which comprises:
a. at least one oligonucleotide probe complementary to at least a part of the mRNA produced by the said non-human organisms cells or genetic assemblies in response to said chemical, mixture of chemicals or other external factors, and labelled with a chemiluminescent acridinium ester; and
b. non-human organisms, cells or genetic assemblies comprising either:
i. a gene responsive to said chemical, mixture of chemicals or other external factor to produce a change in the level of transcribed mRNA as a result of activation or deactivation of said gene; or
ii. a transcription pathway responsive to said chemical, mixture of chemicals or other external factor to produce a change in the level of transcribed mRNA as a result of exposure of said non-human organisms, cells or genetic assemblies to said chemical, mixture of chemicals or other external factors.

## Patentansprüche

1. Verfahren zur Quantifizierung einer Chemikalie, Mischung von Chemikalien oder anderen externen Faktoren, umfassend:
a. Aussetzen gegenüber der Chemikalie, Mischung von Chemikalien oder dem sonstigen externen Faktor eines nicht-menschlichen Organismus, einer Zelle oder genetischen Einheit, der bzw. die ein Gen umfasst, die auf die Chemikalie, Mischung von Chemikalien oder den anderen externen Faktor reagiert, so dass eine Änderung in der Höhe der transkribierten mRNA als Ergebnis von der Aktivierung oder Deaktivierung des Gens auftritt;
b. direktes Binden an die transkribierte mRNA einer Oligonucleotidsonde, die mindestens zu einem Teil der erzeugten mRNA komplementär ist und die mit einem chemilumineszenten Acridiniumester markiert ist; und
c. Quantifizieren der mRNA unter Verwendung eines chemilumineszenten Signals, das durch die Sonde erzeugt wird.

2. Verfahren zur Quantifizierung einer Chemikalie, Mischung von Chemikalien oder anderen externen Faktoren, umfassend:
a. Aussetzen gegenüber der Chemikalie, Mischung von Chemikalien oder dem sonstigen externen Faktor eines nicht-menschlichen Organismus, einer Zelle oder genetischen Einheit, der bzw. die einen Transkriptionssignalweg umfasst, die auf die Chemikalie, Mischung von Chemikalien oder den anderen externen Faktor reagiert, so dass eine Änderung in der Höhe der transkribierten mRNA als Ergebnis des Aussetzens auftritt;
b. direktes Binden an die transkribierte mRNA einer Oligonucleotidsonde, die mindestens zu einem Teil der erzeugten mRNA komplementär ist und die mit einem chemilumineszenten Acridiniumester markiert ist; und
c. Quantifizieren der mRNA unter Verwendung eines chemilumineszenten Signals, das durch die Sonde erzeugt wird.

3. Verfahren nach einem der vorhergehenden Ansprüche, bei dem die chemische Reaktivität des Moleküls, das die Oligonucleotidsiondenmarkierung umfasst, verschieden ist, wenn die Oligonukleotidsonde an die Ziel-mRNA-Sequenz gebunden ist, verglichen mit derjenigen der Oligonukleotidsondenmarkierung, wenn die Oligonukleotidsonde nicht so gebunden ist.

4. Verfahren nach einem der vorhergehenden Ansprüche, bei dem eine Vielzahl von verschiedenen markierten Oligonukleotidsonden verwendet wird, um das Vorhandensein oder das Fehlen von komplementären ZielmRNA-Sequenzen in der gleichen Probe zu erfassen.

5. Verfahren nach einem der vorhergehenden Ansprüche, wobei das Gen ein rekombinantes Molekül in einer prokaryotischen Zelle ist, das mindestens jeweils eine reagierende Elementfunktionalität, die mit einem Reporterelement verknüpft ist, umfasst.

6. Kit, geeignet für das Verfahren nach einem der Ansprüche 1 -5 zur direkten Quantifizierung einer Chemikalie, Mischung von Chemikalien oder anderen externen Faktoren zur Verwendung mit nicht-menschlichen Organismen, Zellen oder genetischen Einheiten, die zuvor der Chemikalie, der Mischung von Chemikalien oder anderen externen Faktoren ausgesetzt wurden, umfassend:
a. mindestens eine Oligonukleotidsonde, die zu mindestens einem Teil der mRNA, die durch die nicht-menschlichen Organismen, Zellen oder genetischen Einheiten in Reaktion auf die Chemikalie, Mischung von Chemikalien oder anderen externen Faktoren erzeugt wurde, komplementär ist und mit einem chemilumineszenten Acridiniumester markiert ist; und
b. nicht-menschliche Organismen, Zellen oder genetische Einheiten, umfassend entweder:
i. ein Gen, das auf die Chemikalien, Mischung von Chemikalien oder den sonstigen externen Faktor reagiert, um eine Änderung in der Höhe der transkribierten mRNA als Ergebnis der Aktivierung oder Deaktivierung des Gens zu bewirken; oder
ii. einen Transkriptionssignalweg der auf die Chemikalie, Mischung von Chemikalien oder den anderen externen Faktor reagiert, um eine Änderung in der Höhe der transkribierten mRNA als Ergebnis des Aussetzens der nicht-menschlichen Organismen, Zellen oder genetischen Einheiten gegenüber der Chemikalie, Mischung von Chemikalien oder den sonstigen externen Faktoren zu bewirken.

## Revendications

1. Procédé de quantification d'un produit chimique, d'un mélange de produits chimiques ou d'autres facteurs externes, comprenant :
a. l'exposition audit produit chimique, mélange de produits chimiques ou autre facteur externe d'un organisme, d'une cellule ou d'un ensemble génétique non humain, comprenant un gène réactif audit produit chimique, mélange de produits chimiques ou autre facteur externe, de sorte qu'un changement a lieu dans le niveau d'ARNm transcrit du fait de l'activation ou de la désactivation dudit gène ;
b. la liaison directe audit ARNm transcrit d'une sonde oligonucléotidique qui est complémentaire d'au moins une partie dudit ARNm produit et qui est marquée par un ester d'acridinium chimioluminescent ; et
c. la quantification dudit ARNm à l'aide d'un signal chimioluminescent produit par ladite sonde.

2. Procédé de quantification d'un produit chimique, d'un mélange de produits chimiques ou d'autres facteurs externes, comprenant :
a. l'exposition audit produit chimique, mélange de produits chimiques ou autre facteur externe d'un organisme, d'une cellule ou d'un ensemble génétique non humain, comprenant une réponse de voie de transcription audit produit chimique, mélange de produits chimiques ou autre facteur externe, de sorte qu'un changement a lieu dans le niveau d'ARNm transcrit du fait de ladite exposition ;
b. la liaison directe audit ARNm transcrit d'une sonde oligonucléotidique qui est complémentaire d'au moins une partie dudit ARNm produit et qui est marquée par un ester d'acridinium chimioluminescent; et
c. la quantification dudit ARNm à l'aide d'un signal chimioluminescent produit par ladite sonde.

3. Procédé selon l'une quelconque des revendications précédentes, dans lequel la réactivité chimique de la molécule contenant le traceur de sonde oligonucléotidique est différent lorsque la sonde oligonucléotidique est liée à la séquence cible de l'ARNm par rapport à celle du traceur de sonde oligonucléotidique lorsque ladite sonde oligonucléotidique n'est pas liée ainsi.

4. Procédé selon l'une quelconque des revendications précédentes, dans lequel une pluralité de sondes oligonucléotidiques marquées différentes servent à détecter la présence ou l'absence de séquences cibles complémentaires d'ARNm dans le même échantillon.

5. Procédé selon l'une quelconque des revendications précédentes, dans lequel le gène est une molécule recombinante dans une cellule procaryote comprenant chacune au moins une fonctionnalité d'élément réactif liée à un élément rapporteur.

6. Kit conçu pour le procédé de l'une quelconque des revendications 1 à 5 pour la quantification directe d'un produit chimique, d'un mélange de produits chimiques ou d'autres facteurs externes utilisés avec des organismes, des cellules ou des ensembles génétiques non humains préalablement exposés audit produit chimique, mélange de produits chimiques ou autres facteurs externes, qui comprend :
a. au moins une sonde oligonucléotidique complémentaire d'au moins une partie de l'ARNm produit par lesdites cellules d'organismes non humains ou par des ensembles génétiques en réponse audit produit chimique, mélange de produits chimiques ou d'autres facteurs externes, et marquée par un ester d'acridinium chimioluminescent ; et
b. des organismes, des cellules ou des ensembles génétiques non humains comprenant soit :
i. un gène réactif audit produit chimique, mélange de produits chimiques ou autre facteur externe, qui produit un changement du niveau d'ARNm transcrit du fait de l'activation ou de la désactivation dudit gène ; ou
ii. une voie de transcription sensible audit produit chimique, mélange de produits chimiques ou autre facteur externe, pour produire une modification du niveau d'ARNm transcrit du fait de l'exposition desdits organismes, cellules ou ensembles génétiques non humains audit produit chimique, mélange de produits chimiques ou autres facteurs externes.
